# EUROPÄISCHE PATENTANMELDUNG

(11) **EP 1 935 353 A1**
(43) Veröffentlichungstag der Anmeldung: **25.06.2008**
(21) Anmeldenummer: 06026780.4
(22) Anmeldetag: 22.12.2006
(51) Int. Cl.: A61B 17/22, A61B 19/00

(54) **Stosswellenpositionierung**

(71) Anmelder: Dornier MedTech Systems GmbH, 82234 Wessling (DE)
(72) Erfinder: Artmeier, Theo, 82194 Gröbenzell (DE)
(74) Vertreter: Grünecker, Kinkeldey, Stockmair & Schwanhäusser Anwaltssozietät

(57) **Zusammenfassung**

Mit der Erfindung soll eine Vorrichtung zum Einleiten von Stoßwellen in den Körper eines Lebewesens, mit einem Therapiekopf, welcher über eine Gelenkanordnung an einem Basisteil der Vorrichtung gelagert und isozentrisch zwangsgeführt ist, dahingehend verbessert werden, dass eine gute isozentrische Zwangsführung des Therapiekopfs sowohl in der Untertisch- als auch in der Obertischposition gewährleistet wird und der Freiraum des Patienten dennoch möglichst wenig eingeschränkt ist. Diese Aufgabe wird durch eine Vorrichtung zum Einleiten von Stoßwellen in den Körper eines Lebewesens gelöst, bei welcher die Gelenkanordnung wenigstens zwei parallelogramartig kinematisch miteinander verbundene Gelenkarme aufweist.

## Beschreibung

Die Erfindung betrifft eine Vorrichtung mit den Merkmalen des Oberbegriffs des Anspruchs 1.

Bei einer aus der DE 39 15 383 bekannten Vorrichtung dieser Gattung ist der Therapiekopf an einem zweiteiligen Gelenkarm gelagert, welcher sich von einer Basiseinheit ausgehend unterhalb einer Patientenliege etwa quer zu dieser erstreckt. Obwohl der erste Teil des Gelenkarms um eine vertikale Achse drehbar an der Basiseinheit gelagert ist, die beiden Teile des Gelenkarms unter einem vorbestimmten Winkel zueinander verdrehbar sind und auch der Therapiekopf selbst drehbar an dem zweiten Teil des Gelenkarms gelagert ist, lässt sich mit dieser Vorrichtung eine Obertischposition nicht erreichen.

In der DE 101 36 177 wird eine gattungsgemäße Vorrichtung beschrieben, bei welcher der Therapiekopf mit einem Röntgen-C-Bogen an einer gemeinsamen Basiseinheit gelagert ist. Der Therapiekopf kann mittels eines Gelenkarms aus einer Park- in eine Behandlungsposition im Bereich einer Patientenliege geschwenkt werden. Über ein Drehgelenk lässt sich der vordere, abgewinkelt ausgebildete Teil des Gelenkarms von einer Untertisch- in eine Obertischposition verschwenken, oder umgekehrt. In der Obertischposition ragt dadurch allerdings der komplette vordere Teil des Gelenkarms über die Patientenliege und den Patienten. Dabei wird sowohl der Bewegungsfreiraum des Patienten als auch der des Arztes eingeschränkt. Viele Patienten empfinden dies zudem als beklemmend.

Der Erfindung liegt die Aufgabe zugrunde, eine Vorrichtung der eingangs genannten Gattung dahingehend zu verbessern, dass eine gute isozentrische Zwangsführung des Therapiekopfes sowohl in der Untertisch- als auch in der Obertischposition gewährleistet wird und der Freiraum des Patienten dennoch möglichst wenig eingeschränkt ist.

Diese Aufgabe wird erfindungsgemäß gelöst mit einer Vorrichtung zum Einleiten von Stoßwellen in den Körper eines Lebewesens mit den Merkmalen des Anspruchs 1.

Durch die wenigstens zwei paralleogrammartig kinematisch miteinander verbundenen Gelenkarme wird der Therapiekopf isozentrisch zwangsführt. Das heißt, die Stoßwellen sind in unterschiedlichen Einstellpositionen des Therapiekopfs stets auf den Focus gerichtet. Zugleich erlaubt die erfindungsgemäße Vorrichtung ein Verschwenken des Therapiekopfs von der Obertisch- in die Untertischposition. So können Konkremente oder erkrankte Körperteile aus beiden Positionen gut erreicht beziehungsweise therapiert werden. Des weiteren erlaubt der parallelogrammartige Hebelmechanismus einen weitgehend uneingeschränkten Freiraum oberhalb der Patientenliege. Je nach Ausführungsform der Erfindung kann das Basisteil, an welchem die Gelenkanordnung gelagert ist, gegebenenfalls unter der Patientenliege angeordnet werden und ermöglicht so, auch bei einem Verschwenken des Therapiekopfs in die Obertischposition eine gute Zugänglichkeit zum Patienten sowie eine gute Bewegungsfreiheit desselben.

In einem günstigen Ausführungsbeispiel der Erfindung können die Anlenkpunkte der Gelenkarme an dem Basisteil im Seitenaufriss auf einer Geraden liegen, welche durch den Focus des Therapiekopfs verläuft. Dies gewährleistet eine gute Isozentrik des Therapiekopfs.

Günstigerweise können die therapiekopfseitigen Anlenkpunkte der Gelenkarme im Seitenaufriss auf einer Geraden liegen, welche durch den Focus des Therapiekopfs verläuft. Auch dies trägt zu einer guten Isozentrik des Therapiekopfs bei.

In einer Variante der Erfindung kann jeder Gelenkarm mindestens ein erstes und mindestens ein zweites Segment aufweisen, welche über ein Gelenk miteinander verbunden sind. Dies erlaubt ein Verschwenken des Therapiekopfs aus der Untertisch- in die Obertischposition und wieder zurück.

Insbesondere können die ersten Segmente der Gelenkarme im Seitenaufriss stets parallel zu einer Geraden verlaufen, welche durch die Anlenkpunkte der Gelenkarme an dem Basisteil und den Focus des Therapiekopfs verläuft. Dies gewährleistet eine gute isozentrische Führung des Therapiekopfs.

Bei einer weiteren Ausführungsform der Erfindung können die zweiten Segmente der Gelenkarme im Seitenaufriss stets parallel zu einer Geraden verlaufen, welche durch die therapiekopfseitigen Anlenkpunkte der Gelenkarme und den Focus des Therapiekopfs verläuft. Dadurch wird ebenfalls die isozentrische Positionierung des Therapiekopfs unterstützt.

Vorteilhafterweise können die Gelenkarme vertikal versetzt zueinander angeordnet sein. Dies ermöglicht eine parallelogrammartige, kinematische Verbindung der Gelenkarme und damit die Ausbildung eines Hebelmechanismus.

In einem günstigen Ausführungsbeispiel der Erfindung kann ein erstes Segment des einen Gelenkarms mit einem zweiten Segment des anderen Gelenkarms überlappend angeordnet sein. Dadurch ergibt sich eine parallelogrammartige Struktur, welche eine gute Führung des Therapiekopfs erlaubt.

In einer Variante der Erfindung können die Gelenkarme in ihrem Überlappungspunkt drehbar miteinander verbunden sein. Dadurch wird eine gute Stabilität und Funktionalität des parallelogrammartigen Hebelmechanismus gewährleistet.

Vorteilhafterweise kann das erste Segment des oberen Gelenkarms mehrteilig sein. Die Einzelteile des ersten Segments des oberen Gelenkarms können so an unterschiedlichen Seiten des unteren Gelenkarms angelenkt sein. Dies gewährleistet einen größeren Bewegungsbereich des Therapiekopfs und erlaubt zudem ein platzsparendes Hintereinanderschieben der Gelenkarme.

In einer Variante der Erfindung können die Einzelteile des ersten Segments fest miteinander verbunden sein. Dadurch wird eine gute Kraftübertragung gewährleistet. Die Einzelteile reagieren somit ähnlich eines einstückig ausgebildeten Segments.

Günstigerweise kann die feste Verbindung über ein Drehgelenk in einem zweiten Segment des unteren Gelenkarms gelagert sein. Dies gewährleistet eine gute Verschwenkbarkeit des Therapiekopfs sowie eine gute Führung des oberen Gelenkarms.

In einer weiteren Ausführungsform der Erfindung können die therapiekopfseitigen Enden der Gelenkarme durch einen Hebelarm verbunden sein. Dies erhöht die Stabilität und gewährleistet eine gute Funktionalität des Hebelmechanismus.

Vorteilhafterweise kann die Längsachse des Hebelarms im Seitenaufriss stets durch den Focus des Therapiekopfs verlaufen. Dies trägt zu einer guten Isozentrik des Therapiekopfs bei.

Im Folgenden wird ein Ausführungsbeispiel der Erfindung anhand der folgenden Zeichnung beschrieben. Dabei zeigen:
- Figur 1: eine erfindungsgemäße Vorrichtung zum Einleiten von Stoßwellen in den Körper eines Lebewesens,
- Figur 2: verschiedene Einstellpositionen des Therapiekopfs der erfindungsgemäßen Vorrichtung,
- Figur 3: eine Gelenkanordnung der erfindungsgemäßen Vorrichtung in perspektivischer Ansicht,
- Figur 4: eine weitere perspektivische Ansicht der erfindungsgemäßen Gelenkanordnung, und
- Figur 5: die erfindungsgemäße Vorrichtung in einer Parkposition.

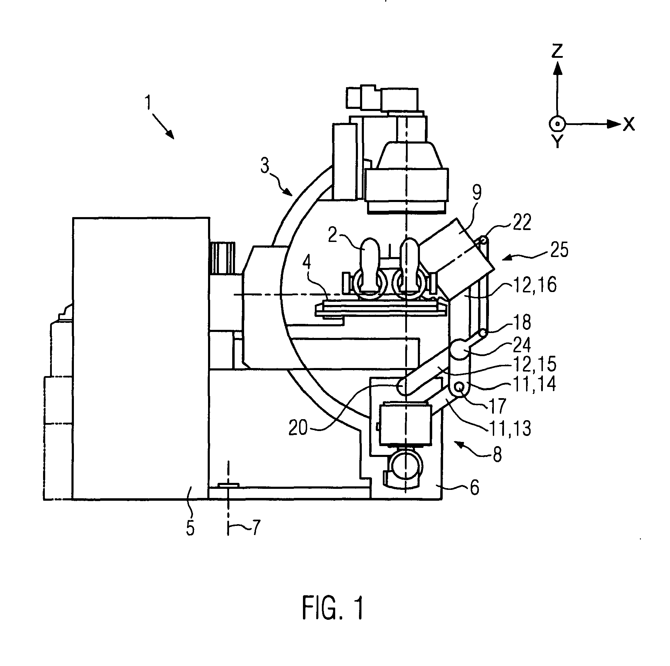

Figur 1 zeigt ein Ausführungsbeispiel einer erfindungsgemäßen Vorrichtung 1 zum Einleiten von Stoßwellen in den Körper eines Lebewesens 2, welches hier durch einen menschlichen Patienten 2 repräsentiert ist. Die Vorrichtung 1 ist hier gemeinsam mit einem Rönten-C-Bogen 3 und einer Patientenliege 4 an einer Basiseinheit 5 gelagert.

Ein Teil der Basiseinheit 5 ist als Fuß 6 ausgebildet. Der Fuß 6 ist um eine vertikale Achse 7 drehbar und trägt an seinem vorderen, der Basiseinheit 5 abgewandten Ende 8 einen Therapiekopf 9. Der Therapiekopf 9 ist mittels einer Gelenkanordnung 10 an dem Fuß 6 der Basiseinheit 5 gelagert. Die Gelenkanordnung 10 weist wenigstens zwei, parallelogrammartig kinematisch miteinander verbundene Gelenkarme 11 und 12 auf. Diese sind etwa parallel und versetzt zueinander angeordnet.

Jeder der Gelenkarme 11, beziehungsweise 12 weist wiederum mindestens zwei hintereinander angeordnete Segmente 13, 14, beziehungsweise 15, 16 auf, welche jeweils über ein Gelenk 17 beziehungsweise 18 miteinander verbunden sind. Ein erstes Segment 13 und ein zweites Segment 14 des Gelenkarms 11 sind über das Gelenk 17 verbunden, während ein erstes 15 und ein zweites Segment 16 des Gelenkarms 12 über das Gelenk 18 verbunden sind. Die Gelenke 17 und 18 sind hier als Drehgelenke ausgebildet. Sie könnten aber auch durch beliebige andere Gelenke, wie z. B. Kugelgelenke ersetzt werden.

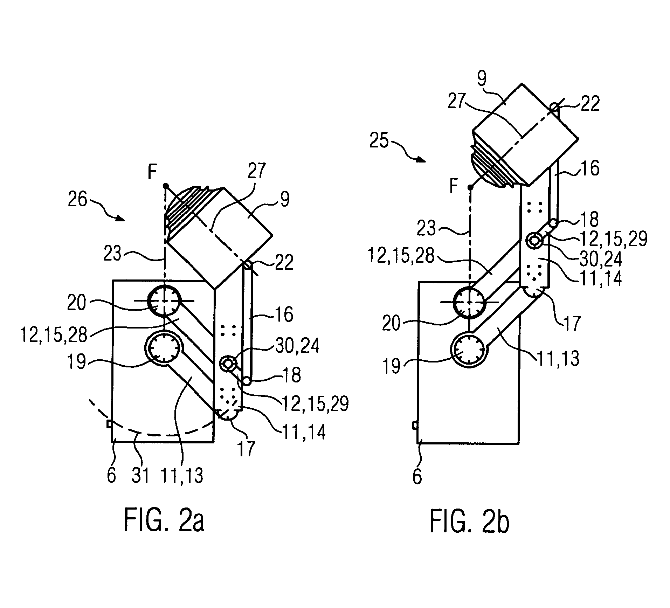

Die Gelenkarme 11 und 12 sind überlappend, beziehungsweise sich überkreuzend angeordnet. In ihrem Überlappungspunkt sind sie durch ein Gelenk 24 verbunden. Diese Konstruktion ermöglicht ein Verschwenken des Therapiekopfs 9 aus der in Figur 1 gezeigten Obertischposition 25 in eine Untertischposition 26 und umgekehrt wie den Figuren 2a bis b dargestellt.

Der Schwenkwinkel, der parallelogrammartigen Gelenkanordnung 10 und damit des Therapiekopfs 9 beträgt in diesem Ausführungsbeispiel 270°. Dadurch kann z. B. sowohl die linke als auch die rechte Niere ohne Umlagern des Patienten mit Stoßwellen behandelt werden. In anderen Ausführungsbeispielen kann der maximal mögliche Schwenkwinkel des Therapiekopfs jedoch auch variieren. Der Schwenkwinkel kann z. B. in einem Bereich von 180° bis 360°, vorzugsweise in einem Bereich von 225° bis 360° und insbesondere in einem Bereich von 270° bis 360° liegen. Alternativ kann der Schwenkwinkel, je nach Anwendungsfall, auch auf einen beliebigen anderen Wert begrenzt werden.

Die Gelenkarme 11, 12 sind jeweils über einen Anlenkpunkt 19, 20 an dem Fuß 6 der Basiseinheit 5 und über je einen Anlenkpunkt 21, 22 an dem Therapiekopf 9 gelagert. In diesem Ausführungsbeispiel sind die Anlenkpunkte 19, 20 und 21, 22 Drehgelenke mit einem rotatorischen Freiheitsgrad. In anderen Ausführungsbeispielen der Erfindung können jedoch auch beliebige andere Gelenke zum Einsatz kommen.

Figur 2 zeigt eine isolierte Ansicht der Gelenkanordnung 10 der erfindungsgemäßen Vorrichtung 1. Die mit 2a bis b bezeichneten Figuren stellen unterschiedliche Einstellposition des Therapiekopfs im Seitenaufriss dar. Die in den Figuren 2a bis b verwendeten Bezugszeichen bezeichnen die selben Teile wie in Figur 1, so dass diesbezüglich auf die Beschreibung der Figur 1 verwiesen wird.

Bei Betrachtung im Seitenaufriss, liegen die Anlenkpunkte 19, 20 der Gelenkarme 11 und 12 auf einer Geraden 23, welche in dieser Ansicht auch durch den Focus F des Therapiekopfs 9 verläuft.

In Relation zu dem Boden, auf welchem der Fuß 6 steht, fällt die Gerade 23 in diesem Ausführungsbeispiel der Erfindung zufällig etwa mit einer Vertikalen zusammen. Im Folgenden wird der Gelenkarm 12 daher als der obere Gelenkarm und der Gelenkarm 11 als der untere Gelenkarm bezeichnet. In anderen Ausführungsbeispielen der Erfindung kann die Gerade 23 jedoch auch von der Vertikalen abweichen.

Auch die therapiekopfseitigen Anlenkpunkte 21, 22 der Gelenkarme 11 und 12 liegen in der Ansicht im Seitenaufriss auf einer Geraden 27, welche ebenfalls durch den Focus F des Therapiekopfs 9 verläuft. Der Focus F bildet somit, bei Betrachtung der Vorrichtung 1 im Seitenaufriss, den Schnittpunkt der beiden Geraden 23 und 27.

Die Figuren 2a bis b zeigen den Therapiekopf 9 in verschiedenen Einstellpositionen. In jeder der hier gezeigten Einstellpositionen verlaufen die zweiten Segmente 14, 16 der Gelenkarme 11 und 12 in etwa parallel zu der Geraden 23 und die ersten Segmente 13 und 15 der Gelenkarme 11, 12 in etwa parallel zu der Geraden 27. Die Geraden 23 und 27 bilden mit den Gelenkarmen 11 und 12 somit stets parallelogrammartige Strukturen.

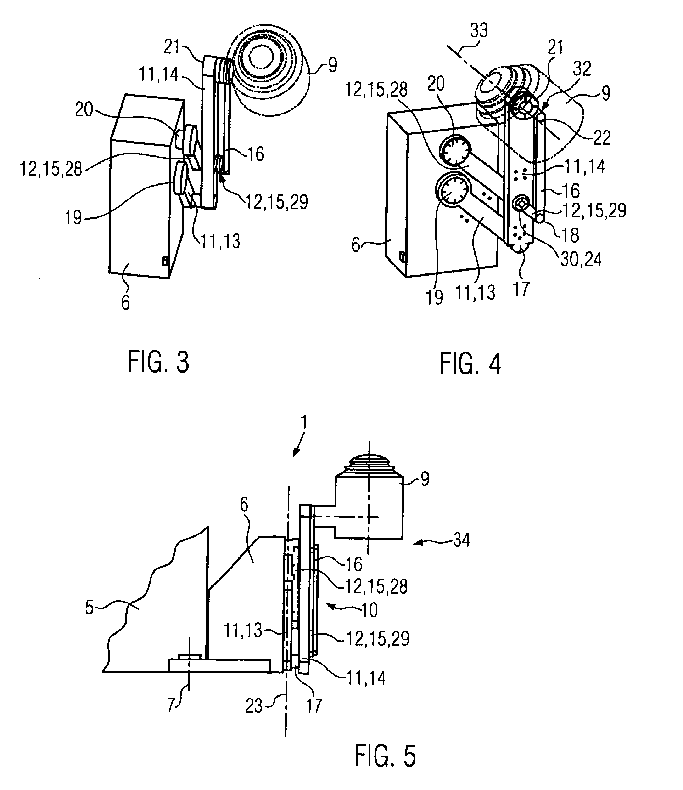

Das erste Segmente 15 des oberen Gelenkarms 12 ist in diesem Ausführungsbeispiel mehrteilig ausgebildet. Es weist hier zwei Einzelteile 28 und 29 auf. Alternativ könnten es, je nach Anwendungsfall, jedoch auch drei oder mehr Einzelteile sein. Die Einzelteile 28, 29 sind über eine feste Verbindung 30, wie z.B. einen Bolzen, verdrehsicher miteinander verbunden. Sie sind auf gegenüberliegenden Seiten des zweiten Segments 14 des unteren Gelenkarms 11 angeordnet, wie z.B. auch in der perspektivischen Ansicht der Figur 3 zu sehen.

Die feste Verbindung 30 der Einzelteile 28 und 29 des ersten Segments 15 des oberen Gelenkarms 12 ist über das Gelenk 24 in dem zweiten Segment 14 des unteren Gelenkarms 11 drehbar gelagert.

Das Einzelteil 28, welches den Anlenkpunkt 20 trägt, ist etwa gleich lang wie das erste Segment 13 des unteren Gelenkarms 11. Durch den vertikalen Versatz der beiden ersten Segmente 13 und 15 beziehungsweise der Anlenkpunkte 19 und 20, befindet sich das Gelenk 24 innerhalb eines durch Drehen des Segments 13 um den Anlenkpunkt 19 beschriebenen Kreises 31, wenn sich der Therapiekopf in einer Untertischposition befindet.

Figur 3 zeigt eine perspektivische Ansicht der Gelenkanordnung 10 aus Figur 1. Die Bezugszeichen der Figur 3 bezeichnen die selben Teile wie in den Figuren 1 bis 2d, so dass diesbezüglich auf die Beschreibung der Figuren 1 bis 2b verwiesen wird.

In der perspektivischen Ansicht der Figur 3 sieht man, dass die Anlenkpunkte 19 und 20 in diesem Ausführungsbeispiel der Erfindung nicht nur vertikal, also entlang der Geraden 23, sondern auch horizontal versetzt zueinander angeordnet sind. Das heißt, dass erstes Segment 15 des oberen Gelenkarms 12 ist von dem Fuß 6 weiter beabstandet, als das erste Segment 13 des unteren Gelenkarms 11. So kann das erste Segment 15 des oberen Gelenkarms 12 an dem ersten Segment 13 des unteren Gelenkarms 11 vorbeigleiten. Auch das erste Segment 13 und das zweite Segment 14 des unteren Gelenkarms 11 sind so weit voneinander beabstandet, dass ein Dazwischenhindurchgleiten des Einzelteils 28 des ersten Segments 15 des oberen Gelenkarms 12 möglich ist.

Figur 4 zeigt eine weitere perspektivische Ansicht der Gelenkanordnung 10. Die in Figur 4 verwendeten Bezugszeichen bezeichnen die selben Teile wie in den Figuren 1 bis 3, so dass diesbezüglich auf die Beschreibung der Figuren 1 bis 3 verwiesen wird.

In Figur 4 ist der Therapiekopf 9 nur durch eine gestrichelte Linie angedeutet. Dadurch wird der Blick auf die therapiekopfseitigen Anlenkpunkte 21 und 22 frei. Der Anlenkpunkt 22 des oberen Gelenkarms 12 und der Anlenkpunkt 21 des unteren Gelenkarms 11 sind in diesem Ausführungsbeispiel der Erfindung durch einen Hebelarm 32 verbunden. Die Längsachse 33 des Hebelarms 32 stimmt in der Ansicht im Seitenaufriss etwa mit der Gerade 27 überein und verläuft in dieser Ansicht ebenfalls durch den Focus F des Therapiekopfs 9.

In diesem Ausführungsbeispiel ist der Hebelarm 32 über den Anlenkpunkt 21 fest mit dem Therapiekopf 9 verbunden, zum Beispiel mittels eines Bolzens. Diese feste Verbindung ist im Anlenkpunkt 21 in dem zweiten Segment 14 des unteren Gelenkarms 11 drehbar gelagert.

Alternativ könnte der Hebelarm 32 auch über beide Anlenkpunkte 21 und 22 fest mit dem Therapiekopf 9 verbunden sein. In diesem Fall müsste auch die zweite feste Verbindung im Anlenkpunkt 22 drehbar in dem zweiten Segment 16 des oberen Gelenkarms 12 gelagert sein. In einer weiteren Alternative der Erfindung kann der Hebelarm 32 auch ganz entfallen. Die Anlenkpunkte 21 und 22 verbinden dann die zweiten Segmente 14, 16 der Gelenkarme 11 und 12 fest mit dem Therapiekopf 9.

Figur 5 zeigt die erfindungsgemäße Vorrichtung 1 in einer Parkposition 34. Die in Figur 5 verwendeten Bezugszeichen bezeichnen dieselben Teile wie in den Figuren 1 bis 4, so dass diesbezüglich auf die Beschreibung der Figuren 1 bis 4 verwiesen wird.

Die Gelenkanordnung 10 ist hier vollständig ineinander geschoben und mit Hilfe des Fußes 6 um die Achse 7 aus dem Behandlungsraum der Patientenliege herausgeschwenkt. Die einander überlappend angeordneten Gelenkarme 11, 12 kommen dabei etwa hintereinander zu liegen. Die einzelnen Segmente 13 bis 16 der Gelenkarme verlaufen annähernd parallel zu der Geraden 23. Bei einer Betrachtung im Seitenaufriß liegen die Gelenkarme 11, 12 etwa auf der Geraden 23.

Im Folgenden wird die Funktionsweise des in den Figuren 1 bis 4 dargestellten erfindungsgemäßen Ausführungsbeispiels erläutert.

Zur Behandlung eines Lebewesens 2 mit Stoßwellen, wird der Therapiekopf 9 durch Schwenken des Fußes 6 der Basiseinheit 5 um die Achse 7 unterhalb der Patientenliege positioniert, auf welcher sich das Lebewesen befindet, wie in Figur 1 dargestellt. Anschließend wird ein Konkrement oder ähnliches in dem Körper des Lebewesens 2 mittels der Ortungseinheit, in diesem Fall des Röntgen-C-Bogens 3, geortet. Dabei wird das Lebewesen mittels der Patientenliege 4 soweit in X-, Y- und Z-Ricntung verfahren bis das Konkrement im Röntgenfokus zu liegen kommt. Der Focus F des Therapiekopfs 9 befindet sich bei dem hier beschriebenen, eingeschwenkten Zustand des Therapiekopfs 9 stets in Übereinstimmung mit dem Röntgenfokus. Alternativ kann der Therapiekopf 9 auch erst nach der Ortung des Konkrements aus seiner Parkposition 34 unter die Patientenliege 4 und damit in eine Behandlungsposition geschwenkt werden, wie in Figur 1 dargestellt.

Im Laufe der Stoßwellenbehandlung kann der Therapiekopf 9 über die Gelenkanordnung 10 isozentrisch, z.B. von einer Untertisch- in eine Obertischposition verschwenkt werden. Dabei bleibt die Lage des Focus F des Therapiekopfs 9 unverändert. Der Winkel und / oder die Richtung in welcher die Stoßwellen in den Körper des Lebewesens 2 eindringen, wird dagegen variiert. So kann das Konkrement abwechselnd von verschiedenen Seiten mit Stoßwellen behandelt werden, während das das Konkrement umgebende gesunde Gewebe so weit wie möglich geschont wird.

Das isozentrische Verschwenken des Therapiekopfes 9 ist in den Figuren 2a bis b dargestellt. Wird der Therapiekopf 9 aus der in Figur 2a dargestellten Untertischposition 26 in die in Figur 2b dargestellte Obertischposition 25 verschwenkt, geschieht folgendes.

Die ersten Segmente 13, 15 des unteren 11 und des oberen Gelenkarms 12 drehen sich um die Anlenkpunkte 19, 20. Das aus mehreren Einzelteilen 28, 29 bestehende erste Segment 15 des oberen Gelenkarms 12 reagiert dabei ähnlich wie ein einstückig ausgebildetes Segment, da die Einzelteile 28, 29 über die Verbindung 30 fest miteinander verbunden sind. Da die beiden ersten Segmente 13, 15 über das zweite Segment 14 des unteren Gelenkarms miteinander verbunden sind, erfolgt die Bewegung dieser beiden Segmente 13, 15 gleichförmig.

Durch die Drehbewegung der ersten Segmente 13, 15 werden auch die Gelenke 17 und 18 sowie die zweiten Segmente 14, 16 des unteren 11 und des oberen Gelenkarms 12 auf jeweils einer Kreisbahn mit nach oben, in Richtung des Therapiekopfs 9 bewegt.

Die versetzte und parallelogrammartige Anordnung der Gelenkarme 11, 12 bildet einen Hebelmechanismus, welcher bei Verdrehen der ersten Segmente 13, 15 um die Anlenkpunkte 19, 20 die Lage des zweiten Segments 16 des oberen Gelenkarms 12 in Relation zu dem zweiten Segment 14 des unteren Gelenkarms 11 verändert. Während beide Segmente 14, 16 eine gleichförmige Aufwärtsbewegung vollführen, wird das Segment 14 zugleich längs des Segments 16 und damit parallel zu der Geraden 23 nach oben, in Richtung des Therapiekopfs 9 verschoben. Dabei ändert sich auch die Position der Anlenkpunkte 21, 22 zueinander, welche an den therapiekopfseitigen Enden der Segmente 14, 16 sitzen.

Da in dem in den Figuren 2a bis b gezeigten Ausführungsbeispiel der Erfindung auch das Gelenk 18, die feste Verbindung 13 und die Anlenkpunkte 21, 22 in etwa ein Parallelogramm bilden, ändert sich mit der Drehbewegung des Segments 15 auch die Lage der Geraden 27 und des Hebelarms 32. Dadurch wird der Therapiekopf 9 um den Focus F gekippt und erreicht so eine Obertischposition. Beim Verschwenken aus der Obertisch- in die Untertischposition erfolgt dieser Bewegungsablauf in umgekehrter Reihenfolge.

Wäre das Segment 15 des oberen Gelenkarms 12 nicht, wie in diesem Ausführungsbeispiel gezeigt, mehrteilig, sondern einstückig ausgebildet, würde die Abwärtsbewegung des Therapiekopfs 9 und der Gelenkanordnung 10 bei der in Figur 2a gezeigten Position enden. Die mehrteilige Ausgestaltung des Segments 15 mit Einzelteilen 28, 29, die auf gegenüberliegenden Seiten des Segments 14 angeordnet sind und die Beabstandung der Anlenkpunkte 19, 20 sowie der Segmente 13 und 14 erlaubt es jedoch, dass die Gelenkanordnung 10 weiter ineinander geschoben werden kann. Hierfür werden die Segmente 13 und 15 in Figur 2a weiter im Uhrzeigersinn um die Anlenkpunkt 19, 20 gedreht. Das Einzelteil 28 des Segments 15 gleitet dabei in der in Figur 2a gezeigten Ansicht vor dem Segment 13 des unteren Gelenkarms 11 vorbei. Dies ist auch in der perspektivischen Ansicht der Figur 3 gut zu sehen. Die zweiten Segmente 14 und 16 der Gelenkarme 11 und 12 folgen dieser Bewegung, bis sie in etwa mit der Geraden 23 übereinstimmen. Das Einzelteil 28 ist nun zwischen dem ersten Segment 13 und dem zweiten Segment 14 des unteren Gelenkarms 11 angeordnet. So kann eine platz- und raumsparende Parkposition 34 des Therapiekopfs 9 erreicht werden, wie sie in Figur 5 dargestellt ist.

## Patentansprüche

1. Vorrichtung (1) zum Einleiten von Stoßwellen in den Körper eines Lebewesens (2), mit einem Therapiekopf (9), welcher über eine Gelenkanordnung (10) an einem Basisteil (6) der Vorrichtung (1) gelagert und isozentrisch zwangsgeführt ist,
**dadurch gekennzeichnet,**
**dass** die Gelenkanordnung (10) wenigstens zwei parallelogrammartig kinematisch miteinander verbundene Gelenkarme (11, 12) aufweist.

2. Vorrichtung (1) nach Anspruch 1,
**dadurch gekennzeichnet,**
**dass** die Anlenkpunkte (19, 20) der Gelenkarme (11, 12) an dem Basisteil (6) im Seitenaufriß auf einer Geraden (23) liegen, welche durch den Fokus (F) des Therapiekopfs (9) verläuft.

3. Vorrichtung (1) nach wenigsten einem der vorhergehenden Ansprüche,
**dadurch gekennzeichnet,**
**dass** die therapiekopfseitigen Anlenkpunkte (21, 22) der Gelenkarme (11, 12) im Seitenaufriß auf einer Geraden (27) liegen, welche durch den Fokus (F) des Therapiekopfs (9) verläuft.

4. Vorrichtung (1) nach wenigsten einem der vorhergehenden Ansprüche,
**dadurch gekennzeichnet,**
**dass** jeder Gelenkarm (11, 12) mindestens ein erstes (13, 15) und mindestens ein zweites Segment (14, 16) aufweist, welche über ein Gelenk (17, 18) miteinander verbunden sind.

5. Vorrichtung (1) nach wenigsten einem der vorhergehenden Ansprüche,
**dadurch gekennzeichnet,**
**dass** die ersten Segmente (13, 15) der Gelenkarme (11, 12) im Seitenaufriß stets parallel zu einer Geraden (23) verlaufen, welche durch die Anlenkpunkte (19, 20) der Gelenkarme (11, 12) an dem Basisteil (6) und den Fokus (F) des Therapiekopfs (9) verläuft.

6. Vorrichtung (1) nach wenigsten einem der vorhergehenden Ansprüche,
**dadurch gekennzeichnet,**
**dass** die zweiten Segmente (14, 16) der Gelenkarme (11, 12) im Seitenaufriß stets parallel zu einer Geraden (27) verlaufen, welche durch die therapiekopfseitigen Anlenkpunkte (21, 22) der Gelenkarme (11, 12) und den Fokus (F) des Therapiekopfs (9) verläuft.

7. Vorrichtung (1) nach wenigsten einem der vorhergehenden Ansprüche,
**dadurch gekennzeichnet,**
**dass** die Gelenkarme (11, 12) vertikal versetzt zueinander angeordnet sind.

8. Vorrichtung (1) nach wenigsten einem der vorhergehenden Ansprüche,
**dadurch gekennzeichnet,**
**dass** sich ein erstes Segment (15) des einen Gelenkarms (12) mit einem zweiten Segment (14) des anderen Gelenkarms (11) überlappend angeordnet ist.

9. Vorrichtung (1) nach wenigsten einem der vorhergehenden Ansprüche,
**dadurch gekennzeichnet,**
**dass** die Gelenkarme (11,12) in ihrem Überlappungspunkt (24) drehbar miteinander verbunden sind.

10. Vorrichtung (1) nach wenigsten einem der vorhergehenden Ansprüche,
**dadurch gekennzeichnet,**
**dass** das erste Segment (15) des oberen Gelenkarms (12) mehrteilig ist.

11. Vorrichtung (1) nach Anspruch 10,
**dadurch gekennzeichnet,**
**dass** die Einzelteile (28, 29) des ersten Segments (15) fest miteinander verbunden sind.

12. Vorrichtung (1) nach Anspruch 11,
**dadurch gekennzeichnet,**
**dass** die feste Verbindung (30) über ein Drehgelenk (24) in dem zweiten Segment (14) des unteren Gelenkarms (11) gelagert ist.

13. Vorrichtung (1) nach wenigsten einem der vorhergehenden Ansprüche,
**dadurch gekennzeichnet,**
**dass** die therapiekopfseitigen Enden (21, 22) der Gelenkarme (11, 12) durch einen Hebelarm (32) verbunden sind.

14. Vorrichtung (1) nach Anspruch 13,
**dadurch gekennzeichnet,**
**dass** die Längsachse (33) des Hebelarms (32) im Seitenaufriß stets durch den Fokus (F) des Therapiekopfs (9) verläuft.
